# EUROPEAN PATENT APPLICATION

(11) **EP 1 306 072 A2**
(43) Date of publication of application: **02.05.2003**
(21) Application number: 02023437.3
(22) Date of filing: 19.10.2002
(51) Int. Cl.: A61K 7/00

(54) **Homogeneous mixtures of silicone oils and organic oils**

(30) Priority: 24.10.2001 US 1293
(71) Applicant: CLARIANT INTERNATIONAL LTD., 4132 Muttenz (CH)
(72) Inventor: Klug, Peter, Dr., 63762 Grossostheim (DE); Simsch, Waltraud, 65779 Kelkheim (DE); Legrow, Gary E., Newberry, Florida 32669 (US)
(74) Representative: Mikulecky, Klaus, Dr.

(57) **Abstract**

The invention relates to mixtures comprising
a) at least one silicone oil,
b) at least one organic oil and
c) at least one organomodified silicone chosen from the formulae (1), (2) or (3)

   Me₃SiO-[Si(Me)(R)O]ₓ-SiMe₃ (1)

   Me₃SiO-[Si(OSiMe₃)(R)O]_{y}-SiMe₃ (2)

   Me₃SiO-[[Si(Me₂)O]_{z}[Si(Me)(R)O]ᵥ]-SiMe₃ (3),
wherein R is an alkyl or alkenyl radical having 6 to 40 carbon atoms, a phenyl radical, an alkylaryl radical having 7 to 30 carbon atoms or an arylalkyl radical having 7 to 30 carbon atoms and x is an integer between 1 and 10, y is an integer between 1 and 10 and v, z are integers between 1 and 100, with the proviso that the silicone oils a) are not organomodified silicones c) according to the formulae (1), (2) or (3).

The addition of the organomodified silicones c) to the mixtures permits the compatibilization of the silicone oils a) and organic oils b) and thus the preparation of homogeneous, clear mixtures.

## Description

### FIELD OF THE INVENTION

This invention relates to homogeneous mixtures of silicone oils, organic oils and organomodified silicones, and to cosmetic and pharmaceutical compositions comprising such mixtures.

### BACKGROUND OF THE PRESENT INVENTION

Mixtures of silicone oils and organic oils (e.g. hydrocarbon oils) frequently form the basis of cosmetic and pharmaceutical compositions. In this connection, a significant limitation for the formulator is that when relatively high viscosity silicone oils are used, incompatabilities frequently arise which lead to a reduction in the stability of the compositions (e.g. emulsions). In particular, dimethyl- and diethylpolysiloxanes, cyclomethicones with (C₁-C₂)-alkyl groups and trimethylsiloxysilicates are often incompatible with organic oils.

European Patent 1 069 150 describes oil-in-oil emulsions wherein the hydrocarbon oil phase and the silicone oil phase, immiscible therein, are emulsified in the presence of a silicone polymer comprising dimethylsiloxane, relatively long-chain alkyl, polyether and crosslinking groups.

Japanese Patent 03264510 describes organomodified silicones of the formulae Me₃SiO[Si(Me)(R¹)O]ₘSiMe₃ and R¹Me₂SiO[SiMe₂O]ₙSiMe₂R¹, and also cyclic silicones where R¹ is (C6-C20)-alkyl and m is 1 or 2 and n is 0 or 1, which are highly compatible with hydrocarbons.

United States Patent 5,932,231 and 5,679,822 describe the preparation of alkyl- and aryltrimethicones and the use thereof in cosmetic compositions.
In the preparation of cosmetic and pharmaceutical compositions, the compatibility of the various oil components is decisive for the sensory, care and esthetic properties of the compositions.

### DESCRIPTION OF THE PRESENT INVENTION

It is an object of this invention to provide phase-stable mixtures of silicone oils and organic oils.

Surprisingly, it has now been found that certain organomodified silicones are highly suitable for the compatibilization of silicone oils and organic oils, which permits the preparation of homogeneous, clear oil mixtures of silicone oils and organic oils. In particular, dimethylpolysiloxanes and cyclomethicones can be made compatible with organic oils in this way. Such oil mixtures are phase-stable and have very good sensory and care properties, as a result of which they are highly suitable for use in cosmetic and pharmaceutical compositions.

This invention provides mixtures comprising
at least one silicone oil,
at least one organic oil and
at least one organomodified silicone chosen from the formulae (1), (2) and/or (3)

Me₃SiO-[Si(Me)(R)O]ₓ-SiMe₃ (1)

Me₃SiO-[Si(OSiMe₃)(R)O]_{y}-SiMe₃ (2)

Me₃SiO-[[Si(Me₂)O]_{z}[Si(Me)(R)O]ᵥ]-SiMe₃ (3),

wherein R is an alkyl or alkenyl radical having 6 to 40 carbon atoms, a phenyl radical, an alkylaryl radical having 7 to 30 carbon atoms or an arylalkyl radical having 7 to 30 carbon atoms and x is an integer between 1 and 10, y is an integer between 1 and 10 and v, z are integers between 1 and 100, with the proviso that the silicone oils a) are not organomodified silicones c) according to the formulae (1), (2) or (3). In the formulae (1), (2) and (3), the alkyl groups may be linear or branched, and saturated or unsaturated. Me is a methyl radical.

The mixtures may comprise various silicones c) of the formulae (1), (2) or (3) and mixtures thereof, where the radicals R can be chosen independently of one another.

The silicone oils a) are advantageously those which do not form homogeneous, clear mixtures with the organic oils b).

The silicone oils a) are preferably dimethylpolysiloxanes, diethylpolysiloxanes, dimethylethylpolysiloxanes, cyclomethicones (cyclic dimethylpolysiloxanes) and cycloethicones (cyclic diethylpolysiloxanes), dimethiconols, diethiconols and trimethylsiloxysilicates, preferably dimethylpolysiloxanes and cyclomethicones.

Preferred dimethylpolysiloxanes and diethylpolysiloxanes are those of the formula R₃SiO[R₂SiO]ₓSiR₃, where R is methyl or ethyl and x is an integer from 2 to 500, e.g. dimethicones of types VICASIL® (General Electric Company), DOW CORNING 200® and DOW CORNING 225 (Dow Corning Corporation).

Preferred dimethiconols and diethiconols are those of the formulae
R₃SiO[SiR₂O]ₓSiR₂(OH) and (HO)R₂SiO[R₂SiO]ₓSiR₂(OH), where R is methyl or ethyl and x is an integer from 1 to 500.

The aforementioned silicone oils a) also include their amino-, fatty-acid-, alcohol-, polyether-, epoxy-, fluorine- and/or alkyl-modified derivatives, and polyether siloxane copolymers, as described in United States 5,104,645 and the publications cited therein, which may be either in liquid form or else in resin form at room temperature.

The organic oils b) may be mineral, animal, vegetable or synthetic oils, preferably hydrocarbons (paraffin oils, vaseline, ceresine, lanolin, purcellin oil, isoparaffin etc.), fluorinated hydrocarbons, perfluorinated hydrocarbons, esters, fatty acid esters, fatty alcohols, triglycerides, monoglycerides and/or sugar esters.

The organic oils are preferably aliphatic or cycloaliphatic hydrocarbons, optionally substituted, preferably having 7 to 40 carbon atoms, particularly preferably dodecane, isododecane, cholesterol, lanolin, hydrogenated polyisobutylenes, docosanes, hexadecane, isohexadecane and paraffins; synthetic oils, such as purcellin oil and isoparaffins; triglycerides of an animal or vegetable origin, preferably beef tallow, pig fat, goose grease, perhydrosqualene, lanolin, sunflower oil, maize oil, soybean oil, rice oil, jojoba oil, babussu oil, pumpkin oil, grapeseed oil, sesame oil, walnut oil, apricot oil, macadamia oil, avocado oil, sweet almond oil, lady's smock oil, castor oil, olive oil, groundnut oil, rapeseed oil and coconut oil; linear and branched fatty alcohols and fatty acid esters, preferably Guerbet alcohols having 6 to 18, preferably 8 to 10, carbon atoms; esters of linear (C₆-C₁₃)-fatty acids with linear (C₆-C₂₀)-fatty alcohols; esters of branched (C₆-C₁₃)-carboxylic acids with linear (C₆-C₂₀)-fatty alcohols; esters of linear (C₆-C₁₈)-fatty acids with branched alcohols, preferably 2-ethylhexanol; esters of linear and branched fatty acids with polyhydric alcohols (e.g. dimerdiol or trimerdiol) and Guerbet alcohols; esters of (C₁-C₁₀)-carboxylic acids or (C₂-C₃₀)-dicarboxylic acids; dioctyl adipate, diisopropyl dimer dilinoleate; propylene glycol/dicaprylate; waxes, preferably beeswax, paraffin wax and microcrystalline waxes, optionally in combination with hydrophilic waxes, such as, for example, cetylstearyl alcohol. Fluorinated and perfluorinated oils; monoglycerides of (C₁-C₃₀)-carboxylic acids; diglycerides of (C₁-C₃₀)-carboxylic acids; triglycerides of (C₁-C₃₀)-carboxylic acids, preferably triglycerides of caprylic/capric acids; ethylene glycol monoesters of (C₁-C₃₀)-carboxylic acids; ethylene glycol diesters of (C₁-C₃₀)-carboxylic acids; propylene glycol monoesters of (C₁-C₃₀)-carboxylic acids; propylene glycol diesters of (C₁-C₃₀)-carboxylic acids; and/or propoxylated and ethoxylated derivatives of the aforementioned classes of compound.
The aforementioned carboxylic acids may contain linear or branched alkyl groups or aromatic groups. Examples, which may be mentioned, are diisopropyl sebacate, diisopropyl adipate, isopropyl myristate, isopropyl palmitate, myristyl propionate, ethylene glycol distearate, 2-ethylhexyl palmitate, isodecyl neopentanoate, di-2-ethylhexyl maleate, cetyl palmitate, myristyl myristate, stearyl stearate, cetyl stearate, behenyl behenate, dioctyl maleate, dioctyl sebacate, cetyl octanoate, diisopropyl dilinoleate, caprylic/capryl triglyceride, PEG-6-caprylic/capryl triglyceride, PEG-8- caprylic/capryl triglyceride, cetyl ricinoleate, cholesterol hydroxy stearate and cholesterol isostearate.

Further suitable organic oils are (C₁-C₃₀)-monoesters and polyesters of glycerol, for example glyceryl tribehenate, glyceryl stearate, glyceryl palmitate, glyceryl distearate and glyceryl dipalmitate.

Likewise suitable as organic oils are (C₁-C₃₀)-carboxylic monoesters and polyesters of sugars, for example glucose tetraoleate, glucose tetraester of soybean oil fatty acid, mannosetetraester of soybean oil fatty acid, galactose tetraester of oleic acid, arabinosetetraester of linoleic acid, xylosetetralinoleate, galactose pentaoleate, sorbitol tetraoleate, sorbitol hexaester of unsaturated soybean oil fatty acid, xylitol pentaoleate, sucrose tetraoleate, sucrose pentaoleate, sucrose hexaoleate, sucrose heptaoleate and sucrose oleate.

Preferred silicones c) are those according to the formulae (1), (2) and/or (3) in which R is an alkyl or alkenyl radical having 6 to 30 carbon atoms, preferably a hexyl radical, caprylyl radical, lauryl radical, palmityl radical, stearyl radical, a (C₂₀-C₂₄) alkyl radical or a (C₂₄-C₂₈) alkyl radical; a phenyl radical; or a phenylmethyl, a phenylethyl, a methylphenyl or an ethylphenyl radical.

Particularly preferred radicals R are hexyl, caprylyl, lauryl, palmityl, stearyl radicals, (C₂₀-C₂₄) alkyl radicals, (C₂₄-C₂₈) alkyl radicals or phenyl radicals, particularly preferably caprylyl and phenyl radicals.

Preferably x is 1, y is an integer between 1 and 5 and z and v are intergers between 1 and 10.

Particularly preferred silicones are alkylmethicones according to formula (1) in which R is an alkyl radical having 6 to 30 carbon atoms, preferably a hexyl radical, a caprylyl radical, a lauryl radical, a palmityl radical, a stearyl radical, a (C₂₀-C₂₄) alkyl radical or a (C₂₄-C₂₈) alkyl radical and x is 1.

Likewise particularly preferred are alkyltrimethicones according to formula (2) in which R is an alkyl radical having 6 to 18 carbon atoms, preferably a hexyl radical, a caprylyl radical, a lauryl radical, a palmityl radical, a stearyl radical, a (C₂₀-C₂₄) alkyl radical or a (C₂₄-C₂₈) alkyl and x is an integer between 1 and 10.

Also particularly preferred are phenyltrimethicones (phenylsequisiloxanes) according to formula (2) in which R is a phenyl radical and y is an integer between 1 and 10.

Also particularly preferred are alkyldimethicone waxes according to formula (3) in which R is an alkyl radical having 18 to 40 carbon atoms and z and v, independently of one another, are a number between 1 and 100.

The weight ratio of silicone oils a) to organic oils b) in the mixtures according to the invention is preferably 100:1 to 1:100, particularly preferably 10:1 to 1:10, especially preferably 5:1 to 1:5.

The weight proportion of silicones c) is, based on the total weight of components a), b) and c), preferably 0.5 to 50% by weight, particularly preferably 2 to 30% by weight, especially preferably 5 to 20% by weight.

The invention also provides for the use of silicones c) chosen from the formulae (1), (2) or (3) for the compatibilization of silicone oils a) and organic oils b) in mixtures comprising silicone oils a) and organic oils b). Here, compabilization is to be understood as meaning that the addition of the silicone c) to a mixture comprising silicone oils a) and organic oils b), which together do not form homogeneous, clear mixtures, leads to the formation of a homogeneous, clear mixture.

The oil mixtures according to the invention display, on the basis of the combination of the property profiles of the silicone oils and organic oils, very favorable sensory, care, esthetic and luster-imparting properties. In terms of performance (ability to be processed, etc.), the oil mixtures likewise have very favorable behavior. Accordingly, the oil mixtures are highly suitable for use in cosmetic and pharmaceutical compositions.

Accordingly, the invention also provides cosmetic and pharmaceutical compositions comprising the oil mixtures according to the invention.

The oil mixtures are suitable for use in rinse-off products, e.g. shampoos, shower preparations, shower gels, foam baths.

The oil mixtures are also suitable for use in leave-on products, e.g. in skincare compositions, day creams, night creams, care creams, nourishing creams, body lotions, ointments, sunscreen compositions, lipcare compositions and deodorants. Furthermore, the oil mixtures are also suitable for surfactant-free emulsions, e.g. for hair cures and hair rinses, hair gels, permanent waving compositions, hair-coloring compositions, and for decorative cosmetics, e.g. make-up, eyeshadow, lipsticks, mascara and the like.

The cosmetic and pharmaceutical compositions according to the invention may comprise, as auxiliaries and additives, all customary anionic, cationic, zwitterionic, nonionic and amphoteric surfactants, oily substances, emulsifiers, coemulsifiers, cationic polymers, film formers, superfatting agents, moisture-donating agents, stabilizers, biogenic active ingredients, glycerol, preservatives, pearlizing agents, dyes and fragrances, solvents, hydrotropic agents, opacifiers, thickeners, dispersants, protein derivatives, such as, for example, gelatin and collagen hydrolyzates, natural- and synthetic-based polypeptides, egg yolk, lecithin, fatty alcohols, deodorizing agents, substances having a keratolytic and keratoplastic action, enzymes and carrier substances, antioxidants, UV light protection filters, pigments and metal oxides, and also antimicrobial agents.

Suitable anionic surfactants are, preferably, (C₁₀-C₂₀)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, α-sulfo fatty acid esters, alkylbenzenesulfonates, alkylphenol glycol ether sulfonates, sulfosuccinates, sulfosuccinic monoesters and diesters, fatty alcohol ether phosphates, protein-fatty acid condensation products, alkyl monoglyceride sulfates and sulfonates, alkyl glyceride ether sulfonates, fatty acid methyltaurides, fatty acid sarcosinates, sulforicinoleates and/or acyl glutamates. These compounds and mixtures thereof are preferably used in the form of their water-soluble or water-dispersible salts, for example the sodium, potassium, magnesium, ammonium, mono-, di- and triethanolammonium salts and analogous alkylammonium salts.

The proportion by weight of the anionic surfactants, based on the finished compositions, is preferably in the range from 2 to 30% by weight, particularly preferably 5 to 25% by weight, especially preferably 12 to 22% by weight.

Preferred cationic surfactants are quaternary ammonium salts, such as
di(C₁₀-C₂₄)alkyldimethylammonium chloride or bromide, preferably di(C₁₂-C₁₈)-alkyldimethylammonium chloride or bromide; (C₁₀-C₂₄)alkyldimethylethylammonium chloride or bromide; (C₁₀-C₂₄)alkyltrimethylammonium chloride or bromide, preferably cetyltrimethylammonium chloride or bromide and (C₂₀-C₂₂)-alkyltrimethylammonium chloride or bromide; (C₁₀-C₂₄)-alkyldimethylbenzylammonium chloride or bromide, preferably (C₁₂-C₁₈)-alkyldimethylbenzylammonium chloride; N-(C₁₀-C₁₈)-alkylpyridinium chloride or bromide, preferably N-(C₁₂-C₁₆)alkylpyridinium chloride or bromide; N-(C₁₀-C₁₈)alkylisoquinolinium chloride, bromide or monoalkyl sulfate;
N-(C₁₂-C₁₈)-alkylpolyoylaminoformylmethylpyridinium chloride; N-(C₁₂-C₁₈)alkyl-N-methylmorpholinium chloride, bromide or monoalkyl sulfate; N-(C₁₂-C₁₈)alkyl-N-ethylmorpholinium chloride, bromide or monoalkyl sulfate; (C₁₆-C₁₈)alkylpentaoxethylammonium chloride; diisobutylphenoxyethoxyethyldimethylbenzylammonium chloride; salts of N,N-diethylaminoethylstearylamide and -oleylamide with hydrochloric acid, acetic acid, lactic acid, citric acid, phosphoric acid;
N-acylaminoethyl-N,N-diethyl-N-methylammonium chloride, bromide or monoalkyl sulfate and N-acylaminoethyl-N,N-diethyl-N-benzylammonium chloride, bromide or monoalkyl sulfate.

The proportion by weight of cationic surfactants, based on the finished compositions, is preferably from 1 to 10% by weight, particularly preferably 2 to 7% by weight, especially preferably 3 to 5% by weight.

Preferred nonionic surfactants are fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acylpolyethylene glycols); polypropylene glycol ethoxylates (Pluronics®); fatty acid alkylolamides, (fatty acid amide polyethylene glycols); N-alkyl-, N-alkoxypolyhydroxy fatty acid amides; sucrose esters; sorbitol esters and polyglycol ethers.

The proportion by weight of nonionic surfactants, based on the finished compositions, is preferably in the range from 1 to 20% by weight, particularly preferably 2 to 10% by weight, especially preferably 3 to 7% by weight.

Preferred amphoteric surfactants are N-(C₁₂-C₁₈)alkyl-β-aminopropionates and N-(C₁₂-C₁₈)alkyl-β-iminodipropionates as alkali metal and mono-, di- and trialkylammonium salts; N-acylaminoalkyl-N,N-dimethylacetobetaines, preferably N-(C₈-C₁₈)acylaminopropyl-N,N-dimethylacetobetaines; (C₁₂-C₁₈)alkyldimethylsulfopropylbetaines; amphoteric surfactants based on imidazoline (trade names Miranol® and Steinapon®), preferably the sodium salt of 1-(β-carboxymethyloxyethyl)-1-(carboxymethyl)-2-laurylimidazolinium; and/or amine oxides, for example (C₁₂-C₁₈)alkyldimethylamine oxide and fatty acid amidoalkyldimethylamine oxide.

The proportion by weight of amphoteric surfactants, based on the finished compositions, is preferably in the range from 0.5 to 20% by weight, particularly preferably 1 to 10% by weight. Furthermore, the compositions according to the invention may comprise foam-boosting cosurfactants from the group of alkylbetaines, alkylamidobetaines, aminopropionates, aminoglycinates, imidazoliniumbetaines and sulfobetaines, amine oxides and fatty acid alkanolamides or polyhydroxyamides.

Particularly preferred surfactants are lauryl sulfate, laureth sulfate, cocamidopropylbetaine, sodium cocoyl glutamate and/or lauroamphoacetate.

A further preferred embodiment relates to leave-on products, in particular emulsions.

The emulsions may either be water-in-oil emulsions or oil-in-water emulsions, microemulsions and multiple emulsions. The emulsions can be prepared in a known manner, i.e. for example by hot, hot/cold or PIT emulsification.

The nonaqueous proportion of the emulsions, which is comprised largely of the emulsifier, the thickener and the oily substance, is usually 5 to 95% by weight, preferably 15 to 75% by weight. Accordingly, the emulsions comprise 5 to 95% by weight, preferably 25 to 85% by weight, of water depending on whether the intention is to prepare lotions with a relatively low viscosity, or creams and ointments with a high viscosity.

Suitable nonionogenic coemulsifiers are, inter alia, addition products of from 0 to 30 mol of ethylene oxide and/or 0 to 5 mol of propylene oxide onto linear fatty alcohols having 8 to 22 carbon atoms, onto fatty acids having 12 to 22 carbon atoms, onto alkylphenols having 8 to 15 carbon atoms in the alkyl group and onto sorbitan or sorbitol esters; also (C₁₂-C₁₈)-fatty acid mono- and diesters of addition products of from 0 to 30 mol of ethylene oxide onto glycerol; glycerol mono- and diesters and sorbitan mono- and diesters of saturated and unsaturated fatty acids having 6 to 22 carbon atoms and optionally ethylene oxide addition products thereof; also addition products of from 15 to 60 mol of ethylene oxide onto castor oil and/or hydrogenated castor oil; polyol and, in particular, polyglycerol esters, such as, for example, polyglycerol polyricinoleate and polyglycerol poly-12-hydroxystearate. Likewise suitable are mixtures of compounds from two or more of these classes of substance.

Examples of suitable ionogenic coemulsifers are anionic emulsifiers, such as mono-, di- or triphosphoric esters, but also cationic emulsifiers, such as mono-, di- and trialkyl quats and polymeric derivatives thereof.

Suitable cationic polymers are those known under the INCI name "Polyquaternium", in particular Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, and Polyquaternium 37&mineral oil&PPG trideceth (Salcare® SC95), PVP dimethylaminoethyl methacrylate copolymer, guar hydroxypropyltriammonium chloride, and calcium alginate and ammonium alginate. It is also possible to use cationic cellulose derivatives; cationic starch; copolymers of diallylammonium salts and acrylamides; quaternized vinylpyrrolidone/vinylimidazole polymers; condensation products of polyglycols and amines; quaternized collagen polypeptides; quaternized wheat polypeptides; polyethyleneimines; cationic silicone polymers, such as, for example, amidomethicones; copolymers of adipic acid and dimethylaminohydroxypropyldiethylenetriamine; polyaminopolyamide and cationic chitin derivatives, such as, for example, chitosan.

Depending on the intended use, suitable film formers are water-soluble polyurethanes, for example C₁₀-polycarbamyl polyglyceryl ester, polyvinyl alcohol, polyvinylpyrrolidone, -copolymers, for example vinylpyrrolldone/vinyl acetate copolymer, water-soluble acrylic acid polymers/copolymers and esters or salts thereof, for example partial ester copolymers of acrylic/methacrylic acid and polyethylene glycol ethers of fatty alcohols, such as acrylate/steareth-20-methacrylate copolymer, water-soluble cellulose, for example hydroxymethylcellulose, hydroxyethylcellulose, hydroxypropylcellulose, water-soluble quaterniums, polyquaterniums, carbocyvinyl polymers, such as carbomers and salts thereof, polysaccharides, for example polydextrose and glucan.

Superfatting agents which may be used are substances such as, for example, polyethoxylated lanolin derivatives, lecithin derivatives, polyol fatty acid esters, monoglycerides and fatty acid alkanolamides, the latter also serving as foam stabilizers. Examples of moisture-donating substances, which are available, are isopropyl palmitate, glycerol and/or sorbitol.

Stabilizers, which can be used, are metal salts of fatty acids, such as, for example, magnesium stearate, aluminum stearate and/or zinc stearate.

Biogenic active ingredients, which can be used, are, for example, plant extracts and vitamin complexes.

In addition, the formulations according to the invention can comprise organic solvents. In principle, suitable organic solvents are all mono- or polyhydric alcohols. Preference is given to using alcohols having 1 to 4 carbon atoms, such as ethanol, propanol, isopropanol, n-butanol, i-butanol, t-butanol, glycerol and mixtures of said alcohols. Further preferred alcohols are polyethylene glycols with a relative molecular mass below 2000. In particular, a use of polyethylene glycol with a relative molecular mass between 200 and 600 and in amounts up to 45% by weight, and of polyethylene glycol with a relative molecular mass between 400 and 600 in amounts of from 5 to 25% by weight is preferred. Further suitable solvents are, for example, triacetin (glycerol triacetate) and 1-methoxy-2-propanol.

The compositions according to the invention can be mixed with conventional ceramides, pseudoceramides, fatty acid N-alkylpolyhydroxyalkylamides, cholesterol, cerebrosides, phospholipids and similar substances as care additive.

In order to adjust the rheological properties of aqueous or solvent-containing emulsions or suspensions, a large number of different systems are given in the specialist literature. Known examples are cellulose ethers and other cellulose derivatives (e.g. carboxymethylcellulose, hydroxyethylcellulose), gelatin, starch and starch derivatives, sodium alginate, fatty acid polyethylene glycol esters, agar agar, tragacanth or dextrins. The synthetic polymers used are various materials, such as, for example, polyvinyl alcohols, polyacrylamides, polyvinylamides, polysulfonic acids, polyacrylic acid, polyacrylates, polyvinylpyrrolidone, polyvinyl methyl ether, polyethylene oxides, copolymers of maleic anhydride and vinyl methyl ether, and various mixtures and copolymers of the abovementioned compounds, including their various salts and esters. These polymers can be crosslinked or uncrosslinked, as desired.

Examples of suitable UV filters are 4-aminobenzoic acid;
3-(4'-trimethylammonium)benzylidenebornan-2-one methylsulfate;
3,3,5-trimethylcyclohexyl salicylate; 2-hydroxy-4-methoxybenzophenone;
2-phenylbenzimidazole-5-sulfonic acid and its potassium, sodium and triethanolamine salts; 3,3'-(1,4-phenylenedimethine)bis-(7,7-dimethyl-2-oxobicyclo[2.2.1]heptane-1-methanesulfonic acid and its salts; 1-(4-tert-butylphenyl)-3-(4-methoxyphenyl)propane-1,3-dione, 3-(4'-sulfo)benzylidenebornan-2-one and its salts; 2-ethylhexyl 2-cyano-3,3-diphenylacrylate; polymer of N-[2(and 4)-(2-oxoborn-3-ylidenemethyl)-benzyl]acrylamide; 2-ethylhexyl 4-methoxycinnamate; ethoxylated ethyl 4-aminobenzoate; isoamyl 4-methoxycinnamate;
2,4,6-tris[p-(2-ethylhexyloxycarbonyl)anilino]-1,3,5-triazine;
2-(2H-benzotriazol-2-yl)-4-methyl-6-(2-methyl-3-(1,3,3,3-tetramethyl-1-(trimethylsilyloxy)disiloxanyl)propyl)phenol; bis(2-ethylhexyl) 4,4'-[(6-[4-((1,1-dimethylethyl)aminocarbonyl)phenylamino]-1,3,5-triazin-2,4-yl)diimino]bis(benzoate);
3-(4'-methylbenzylidene)-D,L-camphor; 3-benzylidenecamphor; 2-ethylhexyl salicylate; 2-ethylhexyl 4-dimethylaminobenzoate; hydroxy-4-methoxybenzophenone-5-sulfonic acid (sulisobenzonum) and the sodium salt; and/or 4-isopropylbenzyl salicylate.

Pigments/micropigments which may be used are, for example, microfine titanium dioxide, mica-titanium oxide, iron oxides, mica-iron oxide, zinc oxide, silicon oxides, ultramarine blue or chromium oxides.

Examples of suitable antioxidants are superoxide dismutase, tocopherol (vitamin E) and ascorbic acid (vitamin C).

Examples of suitable preservatives are phenoxyethanol, parabens, pentanediol or sorbic acid.

Dyes which can be used are the substances approved and suitable for cosmetic purposes.

Suitable fungicidal active ingredients are, preferably, ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine and terbinafine, Zn pyrethione and octopyrox.

The compositions are preferably adjusted to a pH in the range from 2 to 12, particularly preferably pH 3 to 8.

### EXAMPLES

The examples below serve to illustrate the invention, without, however, limiting it thereto. The percentages are percentages by weight, based on the finished mixtures.

Compatibilization of low-viscosity paraffin oil and dimethicone
(viscosity 50 cP, Dow Corning 200 Fluid®)

50 g of Dow Corning 200 Fluid® silicone oil were added to 50 g of low-viscosity paraffin oil. The formation of two immiscible phases was observed. Then, increasing amounts of organomodified silicones c) of the formulae (1), (2) and (3) were added in each case, homogenized by stirring, and the amount of organomodified silicone (in % by weight, based on the total amount of silicone oil and paraffin oil) at which a stable, homogeneous mixture of the oil phases was achieved at room temperature (see table below) was determined.

| Organomodified silicone | % by weight |
|---|---|
| SilCare® 15M40 | 16 |
| SilCare® 15M50 | 15 |
| SilCare® 15M60 | 12 |
| SilCare® 31M30 | 30 |
| SilCare® 31M40 | 24 |
| SilCare® 31M50 | 18.5 |
| SilCare® 31M60 | 11 |
| SilCare® 41M10 | 10 |
| SilCare® 41M15 | 10 |
| SilCare® 41 M20 | 11 |
| SilCare® 41M30 | 17 |
| SilCare® 41 M65 | 17 |

Chemical names of the commercial products used:

| | |
|---|---|
| SilCare® 15M40 | Phenyltrimethicone according to formula (2) where x = 2-3; 200-500 cP |
| SilCare®15M50 | Phenyltrimethicone according to formula (2) where x = 1-3; approx. 20 cP |
| SilCare® 15M60 | Phenyltrimethicone according to formula (2) where x = 1; 5 cP |
| SilCare® 31 M30 | Caprylyltrimethicone according to formula (2) where x = 1-4; 200 - 500 cP |
| SilCare® 31 M40 | Caprylyltrimethicone according to formula (2) where x = 2-3; approx. 50 cP |
| SilCare® 31M50 | Caprylyltrimethicone according to formula (2) where x = 1-3; approx. 50 cP |
| SilCare® 31M60 | Caprylyltrimethicone according to formula (2) where x = 1; 5 cP |
| SilCare® 41M10 | Hexylmethicone according to formula (1) |
| SilCare® 41M15 | Caprylylmethicone according to formula (1) |
| SilCare® 41 M20 | Laurylmethicone according to formula (1) |
| SilCare® 41 M30 | Stearylmethicone according to formula (1) |
| SilCare 41 M65 | Stearyldimethicone according to formula (3), Tₘ = 30 °C |

## Claims

1. A mixture comprising
a) at least one silicone oil,
b) at least one organic oil and
c) at least one organomodified silicone chosen from the formulae (1), (2) or (3)
Me₃SiO-[Si(Me)(R)O]ₓ-SiMe₃ (1)
Me₃SiO-[Si(OSiMe₃)(R)O]_{y}-SiMe₃ (2)
Me₃SiO-[[Si(Me₂)O]_{z}[Si(Me)(R)O]ᵥ]-SiMe₃ (3),
wherein R is an alkyl or alkenyl radical having 6 to 40 carbon atoms, a phenyl radical, an alkylaryl radical having 7 to 30 carbon atoms or an arylalkyl radical having 7 to 30 carbon atoms and x is an integer between 1 and 10, y is an integer between 1 and 10 and v, z are integers between 1 and 100, with the proviso that the silicone oils a) are not organomodified silicones c) according to the formulae (1), (2) or (3).

2. The mixture as claimed in claim 1, wherein the silicone oils a) are dimethylpolysiloxanes, diethylpolysiloxanes, dimethylethylpolysiloxanes, cyclomethicones, cycloethicones, dimethiconols, diethiconols and/or trimethylsiloxysilicates, preferably dimethylpolysiloxanes and/or cyclomethicones.

3. The mixture as claimed in claim 1 or 2, wherein the organic oils b) are hydrocarbons, fluorinated hydrocarbons, perfluorinated hydrocarbons, esters, fatty acid esters, fatty alcohols, triglycerides, monoglycerides and/or sugar esters.

4. The mixture as claimed in anyone of claims 1 to 3, wherein the silicones c) are those according to the formulae (1), (2) or (3) in which R is an alkyl or alkenyl radical having 6 to 30 carbon atoms, a phenyl radical, a phenylmethyl radical, a phenylethyl radical, a methylphenyl radical or an ethylphenyl radical.

5. The mixture as claimed in anyone of claims 1 to 4, wherein R is a hexyl radical, a caprylyl radical, a lauryl radical, a palmityl radical, a stearyl radical, a (C₂₀-C₂₄) alkyl radical, a (C₂₄-C₂₈) alkyl radical or a phenyl radical.

6. The mixture as claimed in anyone of claims 1 to 5, wherein x is 1, wherein y is an integer between 1 and 5 and wherein z and v are intergers between 1 and 10.

7. A cosmetic or pharmaceutical composition comprising the mixture as claimed in anyone of claims 1 to 6, preferably in the form of a rinse-off product or leave-on product.

8. The composition as claimed in claim 7, in the form of an emulsion.

9. The composition as claimed in claim 7 or 8, in the form of a surfactant-free emulsion.

10. The use of organomodified silicones c) chosen from the formulae (1), (2) or (3)
Me₃SiO-[Si(Me)(R)O]ₓ-SiMe₃ (1)
Me₃SiO-[Si(OSiMe₃)(R)O]_{y}-SiMe₃ (2)
Me₃SiO-[[Si(Me₂)O]_{z}[Si(Me)(R)O]ᵥ]-SiMe₃ (3),
wherein R is an alkyl or alkenyl radical having 6 to 40 carbon atoms, a phenyl radical, an alkylaryl radical having 7 to 30 carbon atoms or an arylalkyl radical having 7 to 30 carbon atoms and x is an integer between 1 and 10, y is an integer between 1 and 10 and v and z are integers between 1 and 100, for the compatibilization of silicone oils a) and organic oils b) in mixtures comprising silicone oils a) and organic oils b).
